# EUROPÄISCHE PATENTSCHRIFT

(11) **EP 3 034 044 B1**
(45) Veröffentlichungstag und Bekanntmachung des Hinweises auf die Patenterteilung: **25.10.2017**
(21) Anmeldenummer: 15198672.6
(22) Anmeldetag: 09.12.2015
(51) Int. Cl.: A61F 2/64, A61F 2/68

(54) **HYDRAULISCHER DÄMPFUNGSZYLINDER, INSBESONDERE FÜR EIN PROTHESENKNIEGELENK**
HYDRAULIC SHOCK ABSORBER, ESPECIALLY FOR A PROSTHETIC KNEE JOINT
AMORTISSEUR HYDRAULIQUE, EN PARTICULIER POUR UNE PROTHÈSE DU GENOU

(30) Priorität: 16.12.2014 DE 102014018712; 21.01.2015 DE 102015100876
(43) Veröffentlichungstag der Anmeldung: 22.06.2016
(73) Patentinhaber: Össur Iceland EHF, 110 Reykjavik (IS)
(72) Erfinder: DRESSLER, Janek, 61197 Florstadt (DE); KAMM, Thomas, 63674 Altenstadt (DE); ZOBIREI, Sören, 95463 Bindlach (DE); TSCHUPP, Gabriel, 82256 Fürstenfeldbruck (DE)
(74) Vertreter: Klunker IP Patentanwälte PartG mbB

(56) Entgegenhaltungen:
- EP-A1- 1 736 121
- WO-A1-2010/064063
- DE-U1-202004 008 014
- US-A- 2 478 721
- US-A1- 2013 013 085

## Beschreibung

Die Erfindung betrifft einen hydraulischen Dämpfungszylinder, insbesondere für ein Prothesenkniegelenk, umfassend ein Gehäuse, einen im Gehäuse vorgesehenen und mit einem Hydraulikfluid gefüllten Zylinderraum, und einen in diesem angeordneten Kolben, der über eine in den Zylinderraum eingeführte Kolbenstange im Zylinderraum beweglich ist.

Ein solcher hydraulischer Dämpfungszylinder wird beispielsweise bei einem Prothesenkniegelenk verwendet, um die Flexionsbewegung, bei der das Prothesenkniegelenk, ähnlich wie das menschliche Gelenk, einbeugt, zu dämpfen. Diese Dämpfung erfolgt rein fluidmechanisch, indem die Strömung des aus dem Zylinderraum geförderten Hydraulikfluids in einen Aufnahmeraum entsprechend gedrosselt ist.

Es ist auch bekannt, einen solchen Dämpfungszylinder mit einer Extensionshilfe zu versehen. Eine solche Extensionshilfe stellt einen Energiespeicher dar, der im Rahmen der Flexion geladen wird, und der sich im Rahmen der Extension, wenn also das Prothesenkniegelenk wieder gestreckt wird, entlädt und dabei diese Extensionsbewegung unterstützt. Hierzu ist es bekannt, den Dämpfungszylinder mit einem Federelement auszurüsten, das den Energiespeicher darstellt und das im Rahmen der Flexion gespannt wird und sich bei der Extension wieder entspannt. Die Energiespeicherung ist bei einem solchen Dämpfungszylinder jedoch rein wegabhängig, das heißt, dass das Maß der in der Feder gespeicherten Energie allein davon abhängt, wie stark das Kniegelenk gebeugt wird. Das heißt, dass die gespeicherte Rückstellenergie stets die gleiche ist, unabhängig davon, wie schnell der Prothesenträger läuft, wenn das Prothesenkniegelenk im Rahmen der schnellen oder langsamen Bewegung stets gleichmäßig abgewinkelt wird. Dies ist für den Prothesenträger aber mitunter unangenehm. Denn beim langsamen Laufen ist aufgrund der geringen Schrittfrequenz eine geringere Extensionsunterstützung ausreichend, während beim schnellen Laufen aufgrund der hohen Schrittfrequenz eine stärkere Extensionsunterstützung erforderlich wäre. US2013/0013085 offenbart eine hydraulische Dämpfungsvorrichtung mit zwei Energiespeichervorrichtungen. Der Erfindung liegt damit das Problem zu Grunde, einen demgegenüber verbesserten hydraulischen Dämpfungszylinder anzugeben, der eine geschwindigkeitsabhängige Energiespeicherung ermöglicht.
Zur Lösung des Problems ist bei einem hydraulischen Dämpfungszylinder der eingangs genannten Art erfindungsgemäß vorgesehen, dass im Gehäuse wenigstens zwei separate, unterschiedlich große, mit dem Zylinderraum über Fluidkanäle verbundene Aufnahmeräume für bei einer Kolbenbewegung aus dem Zylinderraum verdrängtes Hydraulikfluid vorgesehen sind, die jeweils über eine Membran mit einem von einem kompressiblen, einen Energiespeicher bildenden Fluid gefüllten Kompressionsraum getrennt sind, wobei dem größeren Aufnahmeraum eine einen Strömungswiderstand für das in den Aufnahmeraum strömende Hydraulikfluid bildende Drosseleinrichtung vorgeschaltet ist, derart, dass das Hydraulikfluid auf die beiden Aufnahmeräume in Abhängigkeit der Geschwindigkeit der Kolbenbewegung verteilbar ist.
Der erfindungsgemäße Dämpfungszylinder zeichnet sich durch zwei unterschiedlich große Aufnahmeräume für das Hydraulikfluid aus, das bei einer Kolbenbewegung aus dem Zylinderraum verdrängt wird. Jedem Aufnahmeraum ist ein separater Kompressionsraum zugeordnet, der mit einem kompressiblen Fluid, üblicherweise ein Gas wie Luft oder Stickstoff, gefüllt ist. Der jeweilige Aufnahmeraum und der jeweilige Kompressionsraum sind über eine flexible Membran voneinander getrennt. Baut sich folglich im Aufnahmeraum durch das einströmende, quasi inkompressible Hydraulikfluid, üblicherweise ein Öl, ein Druck auf, so kommt es zu einer Deformation der Membran und damit zwangsläufig zu einer Verkleinerung des Volumens des Kompressionsraums, was wiederum zu einer Komprimierung des im Kompressionsraum enthaltenen Fluids, also beispielsweise Luft, führt. Dieses komprimierte Fluid bildet den Energiespeicher, die gespeicherte Energie wird im Rahmen der späteren Extensionsbewegung, diese unterstützend, wieder entnommen.

Die beiden Aufnahmeräume sind unterschiedlich groß, können also unterschiedlich viel Hydraulikfluid aufnehmen. Je nachdem, welchem Aufnahmeraum nun eine entsprechende Hydraulikfluidmenge zugeführt wird, wird eine unterschiedlich große Energiemenge im jeweiligen Kompressionsfluid eingespeichert. Um nun die Verteilung des Hydraulikfluids auf die beiden Aufnahmeräume variieren zu können, ist dem größeren Aufnahmevolumen, in das zwangsläufig mehr Hydraulikfluid einströmen muss, um eine vergleichbar große Energiemenge einspeichern zu können, als in den kleineren Aufnahmeraum, eine Drosseleinrichtung vorgeschaltet. Diese Drosseleinrichtung stellt einen definierten Strömungswiderstand für das in den größeren Aufnahmeraum strömenden Hydraulikfluid dar.

Kommt es nun zu einer Flexionsbewegung, so wird Hydraulikfluid aus dem Zylinderraum gedrückt. Bei einer langsamen Bewegung, wenn also das Hydraulikfluid relativ langsam in Richtung der Aufnahmeräume strömt, stellt sich dem Fluidstrom einerseits der Strömungswiderstand der Drosseleinrichtung entgegen, andererseits aber auch der Widerstand, der über die Membran des kleineren Aufnahmeraums gebildet wird. Die Drosseleinrichtung ist dabei bevorzugt so eingestellt, dass der über sie gebildete Strömungswiderstand kleiner ist als der Membranwiderstand der Membran des kleineren Aufnahmeraums. Das Hydraulikfluid strömt demzufolge bevorzugt in den größeren Aufnahmeraum ein, gegebenenfalls zu einem gewissen Anteil natürlich auch in den kleineren Aufnahmeraum. Die dem größeren Aufnahmeraum zugeordnete Membran wird belastet, im zugeordneten Kompressionsraum wird, resultierend aus der Menge an zuströmendem Fluid, im Laufe der langsamen Flexionsbewegung Energie eingespeichert, wobei das Maß an gespeicherter Energie, resultierend aus der Größe des Aufnahmeraums, relativ gering ist.

Wird der über die Drosseleinrichtung gegebene Strömungswiderstand etwas höher eingestellt, so nimmt der Anteil an in den kleineren Aufnahmeraum strömendem Fluid bei langsamer Flexionsbewegung zu, das heißt, dass in Summe gesehen mehr Energie eingespeichert wird. Grundsätzlich sollte jedoch bei sehr langsamer Bewegung der Druck in beiden Aufnahmeräumen synchron steigen.

Bei einer schnelleren Flexionsbewegung hingegen ist der Strömungswiderstand der Drosseleinrichtung höher als der Membranwiderstand der dem kleineren Aufnahmeraum zugeordneten Membran. Das Hydraulikfluid strömt bevorzugt, den Weg des geringeren Widerstands nehmend, in den kleineren Aufnahmeraum und nicht über die Drosseleinrichtung. Dies führt dazu, dass - nachdem das Volumen des kleineren Aufnahmeraums nicht allzu groß ist - eine starke Membrandeformation einsetzt, mithin also das Kompressionsfluid im Kompressionsraum stark komprimiert wird, so dass es zu einem hohen Energieeintrag respektive einer hohen gespeicherten Energiemenge kommt, die im Rahmen der anschließenden Extension zusätzlich bewegungsunterstützend wirkt. Je schneller die Flexionsbewegung also ist, umso größer ist der Anteil des Hydraulikfluids, das in den kleineren Aufnahmeraum strömt, da geschwindigkeitsabhängig der Strömungswiderstand der Drosseleinrichtung wie beschrieben immer größer wird, verglichen mit dem Membranwiderstand. Je schneller folglich die Flexionsbewegung erfolgt, umso größer ist die insbesondere im Energiespeicher, der dem kleineren Aufnahmeraum zugeordnet ist, gespeicherte Energiemenge, die im Rahmen der anschließenden Extensionsbewegung unterstützend abgerufen werden kann.

Das heißt, dass eine automatische und dynamische Ganggeschwindigkeitsanpassung (Autoadaptivität) der Flexions- und Extensionsdämpfung auf rein fluidmechanischer Basis gegeben ist. Der Dämpfungszylinder passt sich also autoadaptiv an die jeweilige Ganggeschwindigkeit an und speichert geschwindigkeitsabhängig unterschiedlich große Energiemengen. Hierdurch kann eine optimale Einstellung der Dämpfung von langsamer Laufgeschwindigkeit (z. B. 2 km/h) bis zu relativ schneller Laufgeschwindigkeit (z. B. 7 km/h) gewährleistet werden, ohne auf eine geschwindigkeitsangepasste Energierückgabe verzichten zu müssen.

Um auf einfache Weise die unterschiedlichen Räume im Gehäuse ausbilden zu können, ist in einer Weiterbildung der Erfindung vorgesehen, dass der Zylinderraum an einem im Gehäuse angeordneten Einsatzbauteil ausgebildet ist. Dieses Einsetzbauteil, das natürlich auch mehrteilig sein kann, bildet zum einen den Zylinderraum. Zum anderen können dann zwischen dem Einsatzbauteil und dem Gehäuse selbst die entsprechenden Aufnahmeräume und Kompressionsräume in Verbindung mit der jeweiligen Membran eingerichtet werden.

Besonders zweckmäßig ist es, wenn der Strömungsquerschnitt der Drossel einstellbar ist, das heißt, dass der Strömungswiderstand, den die Drosseleinrichtung dem Hydraulikfluid entgegensetzt, variiert werden kann. Hierüber kann dann letztlich eine Einstellung erfolgen, wie geschwindigkeitsbezogen die Verteilung des Hydraulikfluids auf die beiden Aufnahmeräume erfolgen soll. Ist beispielsweise der Strömungsquerschnitt der Drosseleinrichtung relativ groß eingestellt, so bedarf es einer sehr hohen Flexionsgeschwindigkeit, damit das Hydraulikfluid zum größten Teil nur in den kleineren Aufnahmeraum strömt. Ist der Strömungsquerschnitt jedoch relativ klein, mithin also der Strömungswiderstand der Drosseleinrichtung hoch, so strömt bereits bei geringerer Flexionsgeschwindigkeit der wesentliche Teil des Hydraulikfluid in den kleineren Aufnahmeraum. Hierüber kann also eine Ganggeschwindigkeitsanpassung in Bezug auf die eingespeicherte Energiemenge erfolgen.

Zur Ermöglichung einer Einstellbarkeit des Strömungsquerschnitts kann die Drosseleinrichtung verstellbares Drosselelement umfassen, über das der Querschnitt einer zum Aufnahmeraum führenden Strömungsbohrung, letztlich also ein Kanalquerschnitt, variierbar ist. Durch einfaches Ein- und Ausschrauben kann folglich die Einstellung erfolgen. Das Drosselelement kann über ein Gewinde beweglich geführt sein, und zur Querschnittsveränderung verschraubt werden. Auch kann das Drosselelement über ein mit einem Gewinde versehenes Einstellstück in seinem Bewegungsumfang limitiert werden.

Zweckmäßigerweise befindet sich die Drosseleinrichtung im Bereich des Bodens des Zylinderraums, wobei auch der größere Aufnahmeraum im Bereich des Zylinderbodens, diesen zumindest abschnittsweise umgebend, vorgesehen ist, während der kleinere Aufnahmeraum oberhalb davon, insbesondere im Bereich der Zylinderdeckelelement vorgesehen ist. Die Aufnahmeräume sind folglich übereinander angeordnet und über entsprechende Fluidkanäle mit dem Zylinderraum verbunden.

Wie beschrieben stellen die beiden Kompressionsräume die Energiespeichermöglichkeit zur Verfügung. Grundsätzlich ist denkbar, jeden Kompressionsraum mit dem kompressiblen Fluid, also beispielsweise Luft oder einem anderen Gas, zu befüllen. Da es sich um ein geschlossenes System handelt, sollte das Fluid nicht entweichen. Um dennoch eine Nachfüllmöglichkeit für den Techniker zu realisieren, bzw. einen gewissen Systemdruck in den Kompressionsräumen je nach Komfortbefinden des Prothesenträgers anzulegen, ist zweckmäßigerweise eine reversibel verschließbare Fluidzuführöffnung vorgesehen, über die kompressibles Fluid in die Kompressionsräume einfüllbar ist. Diese Fluidzuführöffnung ist an der Gehäuseaußenseite vorgesehen und umfasst einen entsprechenden Befüllstutzen, vorzugsweise mit zugeordnetem 2/2-Wege Ventil. Dies ermöglicht eine simultane Befüllung beider Kompressionsräume, wobei der Befülldruck in beiden Räumen gleich ist.

Vorteilhaft ist es ferner, wenn die beiden Kompressionsräume über ein Rückschlagventil miteinander verbunden sind, das bei einem Überdruck im dem größeren Aufnahmeraum zugeordneten Kompressionsraum öffnet. Kommt es beispielsweise beim langsamen Laufen zu einer Befüllung nur des größeren Aufnahmeraums, da der Drosselwiderstand geringer ist als der Membranwiderstand des dem kleineren Aufnahmeraum zugeordneten Kompressionsraums, so steigt mit zunehmender Flexion der Druck im dem größeren Aufnahmeraum zugeordneten Kompressionsraum an. Da dieser Kompressionsraum jedoch nur relativ wenig Energie speichert, da der größere Aufnahmeraum doch ein beachtliches Volumen hat, kann über das Rückschlagventil ein Druckausgleich zum anderen Kompressionsraum erfolgen, so dass auch in diesem Energie gespeichert wird.

Die jeweilige Membran, über die ein Aufnahmeraum vom zugeordneten Kompressionsraum getrennt ist, ist bevorzugt eine Gummi- oder Kunststoffmembran, die hinreichend flexibel ist. Sie ist über entsprechende Befestigungsabschnitte oder Befestigungsmittel gehäuseseitig und, sofern vorgesehen, am Einsatzbauteil abgedichtet befestigt. Da die Aufnahmeräume und Kompressionsräume bevorzugt um den Zylinderraum herumlaufen, ist auch die jeweilige Membran ein ringförmiges Bauteil.

Zweckmäßig ist es, wenn im Rahmen der Flexion auch eine Flexionswegabhängigkeit der Hydraulikfluidzufuhr in die Aufnahmeräume gegeben ist. Denn bei geringer Flexion, beispielsweise einer Flexion bis 20° oder 30°, ist es nicht erforderlich, eine zu große Energie als Extensionsunterstützungsenergie einzuspeichern, da das Kniegelenk ja kaum gebeugt wird. Erst bei größerem Beugungswinkel und damit größerem Flexionsweg ist eine Energieeinspeicherung im größeren Umfang insbesondere über den kleineren Aufnahmeraum wünschenswert. Um dies zu ermöglichen sieht eine zweckmäßige Weiterbildung der Erfindung vor, dass den Aufnahmeräumen ein Schaltventil vorgeschaltet ist, über das der Hydraulikfluidstrom in Abhängigkeit der Position des Kolbens oder der Kolbenstange dem größeren Aufnahmeraum an der Drosseleinrichtung vorbei oder über die Drosseleinrichtung zuführbar ist. Der Flexionsweg geht unmittelbar in die Kolben- oder Kolbenstangenposition ein. Über das erfindungsgemäß vorgesehene Schaltventil ist es nun möglich, positionsabhängig den Hydraulikfluidstrom entweder an der Drosseleinrichtung vorbei und folglich quasi ungedrosselt in den größeren Aufnahmeraum zu führen, oder ihn über die Drosseleinrichtung zu führen. Im erstgenannten Fall strömt das aus dem Zylinderraum verdrängte Hydraulikfluid im Wesentlichen in den größeren Aufnahmeraum, da diesem Zustrom letztlich nahezu kein Strömungswiderstand, abgesehen vom Membranwiderstand des dem größeren Aufnahmeraum zugeordneten Kompressionsraums, entgegengesetzt wird. Dieser Membranwiderstand ist jedoch geringer als der Membranwiderstand des Kompressionsraums, der dem kleineren Aufnahmeraum zugeordnet ist. Das heißt, dass das Hydraulikfluid nahezu vollständig in den größeren Aufnahmeraum einströmt und es hierbei nur zu einer sehr geringen Energieeinspeicherung im zugeordneten Kompressionsraum kommt. Bei einer weiteren Flexionsbewegung jedoch wird das Schaltventil betätigt und der Hydraulikfluidstrom läuft über die Drosseleinrichtung. Nun setzt die geschwindigkeitsabhängige Fluidverteilung ein, wie zuvor beschrieben. Bei langsamer Flexionsgeschwindigkeit ist wie beschrieben der Strömungswiderstand der Drosseleinrichtung geringer als der Membranwiderstand seitens der Membran des dem kleineren Aufnahmeraum zugeordneten Kompressionsraums. Daher strömt das Hydraulikfluid bevorzugt in den größeren Aufnahmeraum. Bei langsamer Bewegung steigt der Druck in beiden Kompressionsräumen nahezu synchron, resultierend auch aus der Druckausgleichsmöglichkeit über das beide Kompressionsräume koppelnde Rückschlagventil. Bei hoher Flexionsgeschwindigkeit jedoch wird der Strömungswiderstand der Drosseleinrichtung deutlich größer als der Membranwiderstand der dem kleineren Aufnahmeraum zugeordneten Membran, das Hydraulikfluid fließt bevorzugt in den kleineren Aufnahmeraum. Mit steigender Geschwindigkeit wird mehr und mehr kinetische Energie im Kompressionsfluid gespeichert. Bei sehr schneller Bewegung ist der Strömungswiderstand der Drosseleinrichtung so groß, dass das Hydraulikfluid fast ausschließlich in den kleineren Aufnahmeraum strömt.

Folglich ist bei Realisierung des Schaltventils sowohl eine Wegabhängigkeit als auch eine Geschwindigkeitsabhängigkeit gegeben.

Das Schaltventil kann dabei ein am Zylinderboden aufstehendes Standrohr umfassen, das vom Kolben und der Kolbenstange übergriffen ist, und das über eine Bohrung mit dem Zylinderraum kommuniziert, so dass Hydraulikfluid durch das Standrohr so lange in den größeren Aufnahmeraum zuführbar ist, bis die Bohrung den in Richtung des Zylinderbodens bewegten Kolben oder die Kolbenstange geschlossen ist, wonach das Hydraulikfluid über die Drosseleinrichtung strömt. Das Schaltventil ist also als 2/2-Wege Ventil ausgebildet. Je nach Position des Kolbens oder der Kolbenstange und damit je nach Flexionswinkel kann das verdrängte Hydraulikfluid entweder durch das Standrohr unmittelbar und unter Umgehung der Drosseleinrichtung oder über die Drosseleinrichtung in den größeren Aufnahmeraum strömen. Bei kleinerem Flexionswinkel strömt das Hydraulikfluid quasi widerstandsfrei durch das Standrohr in den größeren Aufnahmeraum. Bei größerem Flexionswinkel hingegen ist die in das Standrohrinnere führende Bohrung durch den Kolben oder die Kolbenstange geschlossen, so dass das Hydraulikfluid zwangsläufig über die Drosseleinrichtung strömen muss, um in den größeren Aufnahmeraum zu gelangen.

Zweckmäßigerweise greift das Drosselelement in das Standrohr ein, wobei die Strömungsbohrung, die über das Drosselelement in ihrem Strömungsquerschnitt variiert werden kann, im Standrohr ausgebildet ist. Das Drosselelement, das wie beschrieben bevorzugt durch Verschrauben in seiner Position variiert werden kann, und das über das Gewinde im Gehäuse geführt ist, erstreckt sich also von unten in das Standrohr. Es liegt bevorzugt an der Standrohrinnenseite an. Die Strömungsbohrung ist standrohrseitig vorgesehen, so dass je nach Position des Drosselelements die Strömungsbohrung mehr oder weniger weit geöffnet ist. Das in das Standrohr einströmende Hydraulikfluid kann durch das Drosselelement axial gesehen strömen, wozu das Drosselelement eine oder mehrere entsprechende Durchgangsbohrungen aufweist, so dass ein nahezu ungehinderter Zustrom in den größeren Aufnahmeraum möglich ist.

In Weiterbildung der Erfindung kann vorgesehen sein, dass am Kolben ein bei einer Bewegung in Richtung des Zylinderbodens öffnendes Rückschlagventil vorgesehen ist, über das der unterhalb des Kolbens befindliche Teil des Zylinderraums mit dem oberhalb des Kolbens befindlichen Zylinderraum verbindbar ist. Über dieses Rückschlagventil ist es möglich, den Kolben hydraulisch von der Kolbenstange zu entkoppeln. Im Rahmen der Flexionsbewegung taucht die Kolbenstange immer weiter in den Zylinderraum ein. Über das Rückschlagventil kann nun Hydraulikfluid vom unteren Zylinderraumteil in den oberen Zylinderraumteil strömen. Das heißt, dass der Kolben im Rahmen der Flexionsbewegung kein Verdrängerelement darstellt. Die Menge des aus dem Zylinderraum verdrängten Hydraulikfluid ist ausschließlich davon abhängig, wie weit die Kolbenstange in den Zylinderraum eintaucht, das heißt, dass allein die Kolbenstange das Verdrängerelement darstellt.

Zur Ausbildung des Rückschlagventils ist zweckmäßigerweise ein die Kolbenstange umgreifender Dichtring und eine am Kolben vorgesehene Bohrung, die vom Dichtring übergriffen ist, vorgesehen. Der Dichtring hebt sich von seinem die Bohrung verschließenden Sitz am Kolben ab, sobald die Kolbenstange und damit der Kolben im Zylinderraum niedergedrückt wird. Bei einer Bewegung in Gegenrichtung sitzt der Dichtring die Bohrung verschließend am Kolben auf, so dass das Rückschlagventil geschlossen ist.

Bei der Extensionsbewegung wird wie beschrieben der Kolben und die Kolbenstange wieder in Richtung des Zylinderdeckelelements bewegt. Hierbei verkleinert sich der oberhalb des Kolbens liegende Zylinderraumteil. In den unterhalb des Kolbens befindlichen Zylinderraumteil strömt das aus den Aufnahmeräumen zurückgedrückte Hydraulikfluid nach. Um das aus dem sich verkleinernden oberen Zylinderraumteil zu verdrängende Öl abzuführen kann bei einer Weiterbildung der Erfindung ein Drosselventil vorgesehen sein, über das der unterhalb des Kolbens befindliche Teil des Zylinderraums mit dem oberhalb des Kolben befindlichen Teil des Zylinderraums kommuniziert, über welches Drosselventil bei einer Bewegung des Kolbens in Richtung des Zylinderdeckelelements Hydraulikfluid aus dem oberhalb des Kolbens befindlichen Teil in den unterhalb befindlichen Teil strömt, bis es bei Erreichen eines definierten Kolbenposition geschlossen ist. Über dieses Drosselventil ist eine gedämpfte Extensionsbewegung realisiert, da die Geschwindigkeit, mit der die Kolbenstange aus dem Zylinderraum gezogen werden kann, vom Fluidfluss durch dieses Drosselventil beeinflusst ist. Dieser Fluidfluss ist beispielsweise bis zu einer Gelenkbeugung von 15° möglich, dann schließt sich das Drosselventil und die beiden Zylinderraumteile sind wieder voneinander getrennt. Eine weitergehende Bewegung ist dann, wenn nicht eine entsprechende Möglichkeit zur weiteren Hydraulikfluidabfuhr vorgesehen ist, kaum noch möglich. Dabei kann das Drosselventil so ausgestaltet sein, dass sich der Strömungsquerschnitt zunehmend, also nicht abrupt, auf null verringert, so dass eine gewisse Bewegungsdämpfung innerhalb der Extensionsbewegung gegeben ist.

Das Drosselventil kann in Weiterbildung der Erfindung zweckmäßigerweise eine am Standrohr längs laufende Nut und eine kolbenseitig oder kolbenstangenseitig ausgebildete Bohrung umfassen, die bei geöffnetem Drosselventil mit der Nut und dem oberen Teil des Zylinderraums kommuniziert. Über diese Bohrung kann folglich Hydraulikfluid aus dem sich verkleinernden oberen Zylinderraumteil in die standrohrseitig vorgesehene Nut einströmen und in den unteren Zylinderraumteil gelangen. Läuft nun diese Nut an ihrem oberen Ende schräg aus, so kann die zuvor beschriebene Dämpfungsmöglichkeit realisiert werden, da dann mit zunehmendem Herausziehen der Kolbenstange der Strömungsquerschnitt dieses Drosselventils immer weiter abnimmt.

Soll wie beschrieben eine Endlagendämpfung realisiert werden, mithin also der Kolben respektive die Kolbenstange gedämpft und ohne abruptes Anschlagen in eine definierte Endlage bewegbar sein, so sieht die Erfindung die Anordnung wenigstens eines Verstelldrosselventils vor, über das der Strömungsquerschnitt eines Hydraulikfluidkanals, der den oberhalb des Kolbens befindlichen Teil des Zylinderraums mit dem kleineren Aufnahmeraum verbindet, variierbar ist. Dieses Verstelldrosselventil definiert einen sehr kleinen Strömungsquerschnitt seitens des Hydraulikfluidkanals, so dass sehr wenig Hydraulikfluid in den kleineren Aufnahmeraum gelangen kann, aus dem es in den unterhalb des Kolbens befindlichen Zylinderraumteil strömt. Durch den geringen Strömungsquerschnitt ist folglich die weitere Extensionsbewegung bis zur vollständigen Streckung stark gedämpft, so dass die mechanischen Komponenten sehr langsam in die Endlage laufen.

Das Verstelldrosselventil kann beispielsweise eine über ein Gewinde im Gehäuse befindlich geführte Drosselschraube umfassen, über die der Querschnitt des Hydraulikfluidkanals variierbar ist. Bevorzugt sind zwei Verstelldrosselventile vorgesehen, wobei das Verstelldrosselventil in einer festen Stellung, einen Mindestdurchfluss definierend gesichert, insbesondere verplombt ist. Der Techniker kann über das erste Verstelldrosselventil nach Komfortbefinden des Prothesenträgers die Endlagendämpfung einstellen, mithin also durch Verschrauben der Drosselschraube den Strömungsquerschnitt des Hydraulikfluidkanals variieren. Um zu vermeiden, dass versehentlich der Strömungsquerschnitt vollständig geschlossen wird und es in Folge dessen zu keinerlei Bewegung mehr kommt, also der Zylinder quasi gesperrt wäre, ist das zweite Verstelldrosselventil vorgesehen, das einen Mindestdurchfluss definiert und in einer gesicherten Position fixiert ist und vom Techniker oder Anwender nicht betätigt werden kann.

Wie beschrieben erfolgt bei dem Dämpfungszylinder ein Austausch des Hydraulikfluids zwischen dem Zylinderraum und den Aufnahmeräumen. Bei einer Flexionsbewegung wird Hydraulikfluid in die Aufnahmeräume gedrückt, bei einer Extensionsbewegung strömt das Hydraulikfluid aus den Aufnahmeräumen zurück. Dieser Kreislauf umfasst bevorzugt, aber nicht notwendigerweise ein Hydraulikfluidreservoir. Um ein solches zu bilden, ist bevorzugt ein zusätzlicher Aufnahmeraum für bei einer Bewegung des Kolbens in Richtung des Zylinderdeckelelements aus dem größeren Aufnahmeraum zurückströmendes Hydraulikfluid vorgesehen. Dieser zusätzliche Aufnahmeraum ist bevorzugt im Inneren der Kolbenstange realisiert, die über ein Rückschlagventil mit dem Inneren des Standroheres verbunden ist, welches Rückschlagventil bei einer Bewegung durch den anliegenden Hydraulikfluiddruck des vom größeren Aufnahmeraum her in das Standrohr einströmenden Hydraulikfluids öffnet. Im Rahmen der Extensionsbewegung füllt sich folglich der kolbenstangenseitige Aufnahmeraum, während er bei einer Flexionsbewegung entleert wird.

Um diese Entleerung auf einfache Weise zu ermöglichen ist zweckmäßigerweise am Standrohr wenigstens eine im Bereich des oder oberhalb des Rückschlagventils angeordnete Durchbrechung vorgesehen, an der eine an der Außenseite des Standrohrs ausgebildete, sich in Richtung des Zylinderbodens erstreckende Nut mündet. Im Rahmen der Flexion, wenn die Kolbenstange in den Zylinderraum eintaucht und folglich über das Standrohr läuft, kommt es zwangsläufig zu einer Volumenverkleinerung des Aufnahmeraums im Kolbenstangeninneren. Das Hydraulikfluid kann nicht über das gesperrte Rückschlagventil fließen. Stattdessen strömt es durch die eine oder die mehreren standrohrseitig vorgesehenen Durchbrechungen am Rückschlagventil vorbei in entsprechende standrohraußenseitig ausgebildete Nuten, von denen aus es unabhängig von der Position der Kolbenstange relativ zum Rückschlagventil respektive Standrohr in den unteren Zylinderraumteil strömen kann. Vorzugsweise sind zwei einander gegenüberliegende Durchbrechungen und entsprechende einander gegenüberliegende Nuten vorgesehen.

Neben dem Dämpfungszylinder selbst betrifft die Erfindung ferner ein Prothesenkniegelenk, umfassend einen Dämpfungszylinder der beschriebenen Art.

Weitere Vorteile und Einzelheiten der Erfindung ergeben sich aus dem im Folgenden beschriebenen Ausführungsbeispiel sowie anhand der Zeichnungen. Dabei zeigen:
- Figur 1: eine Schnittansicht durch einen erfindungsgemäßen hydraulischen Dämpfungszylinder,
- Figur 2: ein hydraulisches und pneumatisches Blockschaltbild,
- Figur 3: eine Teilansicht des Dämpfungszylinders aus Figur 1 zur Darstellung der Aufnahme- und Kompressionsräume,
- Figur 4: eine Ansicht entsprechend Figur 3 zu Beginn der Flexionsbewegung mit geöffnetem Schaltventil,
- Figur 5: eine Ansicht entsprechend Figur 4 bei fortgesetzter, langsamer Flexionsbewegung mit geschlossenem Schaltventil,
- Figur 6: eine Ansicht entsprechend Figur 5 bei schneller Flexionsbewegung mit geschlossenem Schalventil,
- Figur 7: eine Ansicht ausgehend von Figur 6 zu Beginn der Extensionsbewegung,
- Figur 8: eine Ansicht bei fortgesetzter Extensionsbewegung und Einlaufen in den Endanschlag, und
- Figur 9: eine Seitenansicht des Standrohres des Dämpfungszylinders zur Darstellung der Durchbrechungen und außenseitigen Nuten.

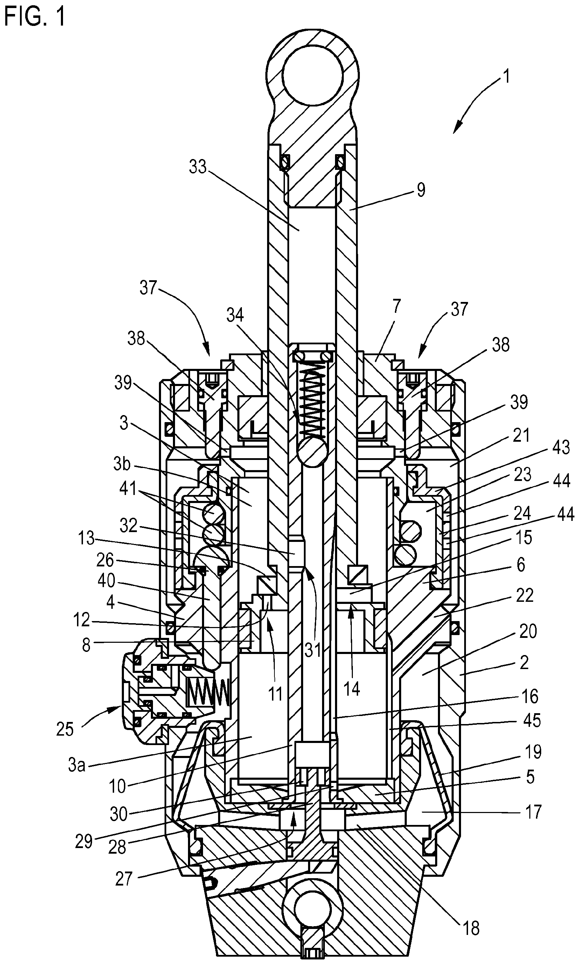

Figur 1 zeigt einen erfindungsgemäßen hydraulischen Dämpfungszylinder 1, der für ein Prothesenkniegelenk geeignet ist. Er umfasst ein Gehäuse 2, in dessen Innerem ein Einsatzbauteil 4 angeordnet ist, das einen Zylinderraum 3 aufweist. Das Einsatzbauteil 4 ist mehrteilig, es umfasst einen Zylinderboden 5 sowie einen den Zylinderraum 3 radial abschließenden Bauteilkörper 6. Oberseitig ist der Zylinderraum 3 über ein Zylinderdeckelelement 7, das gehäuseseitig festgelegt ist, geschlossen.

Im Inneren des Zylinderraums 3 ist ein Kolben 8 vorgesehen, der mit einer Kolbenstange verbunden ist. Der Kolben 8 und die Kolbenstange 9 laufen respektive umgreifen ein Standrohr 10, das am Zylinderboden 5 befestigt ist. Der Kolben 10 trennt den Zylinderraum 3 in einem unteren Zylinderraumteil 3a und einen oberen Zylinderraumteil 3b. Kolbenseitig und kolbenstangenseitig ist ein Rückschlagventil 11 vorgesehen, realisiert über eine kolbenseitige Bohrung 12 sowie einen kolbenstangenseitig angeordneten Dichtring 13. Das Rückschlagventil 11 öffnet, wenn der Kolben in Richtung des Zylinderbodens 5 bewegt wird. Es schließt bei einer Bewegung in entgegengesetzte Richtung. Ferner ist ein Drosselventil 14 vorgesehen umfassend eine kolbenstangenseitig vorgesehene Bohrung 15 sowie eine entlang der Standrohraußenseite vorgesehene, längs laufende Nut 16, die sich, siehe Figur 1, nach oben hin verjüngt.

Zwischen Gehäuse 2 und Einsatzbauteil 4 sind verschiedene Aufnahme- und Kompressionsräume gebildet. Vorgesehen ist ein erster größerer Aufnahmeraum 17, der im Bereich des Zylinderbodens 5 vorgesehen ist und sich ringförmig um das Einsatzbauteil 4 erstreckt. In ihn mündet ein oder münden mehrere Fluidkanäle 18, die mit dem Zylinderraum 3 kommunizieren. Der größere Aufnahmeraum 17 ist über eine Membran 19 von einem ersten Kompressionsraum 20 getrennt. Im Kompressionsraum 20 ist ein kompressibles Fluid, beispielsweise Luft oder ein anders Gas, aufgenommen. Durch Einströmen des Hydraulikfluid, das im Zylinderraum 3 ist, in den Aufnahmeraum 17 wird die flexible Membran 19, eine Gummi- oder Kunststoffmembran, deformiert und das Volumen des Kompressionsraums 20 verkleinert. Damit einher geht eine Kompression des Kompressionsfluids und damit eine Energiespeicherung.

Des Weiteren vorgesehen ist ein kleinerer Aufnahmeraum 21, der im Bereich des oberen Zylinderendes vorgesehen ist. Der Aufnahmeraum 21 ist von dem Zylinderraum 3 über einen oder mehrere Hydraulikkanäle 22 verbunden. Ihm zugeordnet ist ein zweiter Kompressionsraum 23, der ebenfalls über eine flexible Membran 24 vom Aufnahmeraum 21 getrennt ist. Die Membran 24 ist in einer Manschette 43 übergriffen, die mit Löchern 44 perforiert ist, über die das Hydraulikfluid an der Membran 24 anliegt. Auch in diesem Kompressionsraum 23 befindet sich ein Kompressionsfluid, beispielsweise ebenfalls Luft oder ein Gas. Der Aufnahmeraum 21 ist kleiner als der Aufnahmeraum 17. Auch kann die Membran 24 etwas steifer sein als die Membran 19, mithin also einen größeren Membranwiderstand besitzen. Wird Hydraulikfluid aus dem Zylinderraum 3 in den Aufnahmeraum 21 befördert, so kommt es auch dort druckbedingt zu einer Deformation der Membran 24 und damit zu einer Verkleinerung des Kompressionsraumvolumens und mithin zu einer Kompression des dortigen Fluids. Auch hierüber erfolgt eine Energieeinspeicherung im Kompressionsfluid.

Die Kompressionsräume 20 und 23 können über eine Fluidzuführöffnung 25 mit dem Kompressionsfluid befüllt werden. Die Fluidzuführung 25 ist als Befüllstutzen mit einem 2/2-Wege Ventil ausgeführt. Vorgesehen ist des Weiteren ein Rückschlagventil 26, das einen Druckausgleich im Fall eines Überdrucks im Kompressionsraum 20 zum Kompressionsraum 23 ermöglicht. Auch die Kompressionsräume 20 und 23 wie auch die Aufnahmeräume 21 und 17 laufen ringförmig um das Einsatzbauteil 4, auch die Membranen 19 und 24 sind entsprechend ringförmige Bauteile.

Vorgesehen ist des Weiteren eine Drosseleinrichtung 27 umfassend ein Drosselelement 28, das über eine Gewindeverbindung am Gehäuse 2 oder am Standrohr 10 verstellbar geführt ist. Es greift wie Figur 1 zeigt in das Standrohr 10 ein. Die Drosseleinrichtung 27 umfasst ferner eine Strömungsbohrung 29, die am Standrohr 10 ausgebildet ist, und deren Strömungsquerschnitt durch entsprechende Positionierung des Drosselelements 28 entsprechend variiert werden kann. Über die Drosseleinrichtung 27 kann folglich der freie Strömungsquerschnitt zum größeren Aufnahmeraum 17 verstellt werden und folglich ein variierbarer Strömungswiderstand für aus dem Zylinderraum 3 kommendes Hydraulikfluid zum Aufnahmeraum 17 eingestellt werden.

Das Drosselelement 28 weist mehrere Längsbohrungen 30 auf, die es ermöglichen, dass in das Innere des Strömungsrohres 10 einströmendes Hydraulikfluid unter Umgehung der eigentlichen Drossel, hier also der im Strömungsquerschnitt reduzierten Strömungsbohrung 29 in den Aufnahmeraum 17 strömen kann.

Damit Hydraulikfluid in das Innere des Strömungsrohres 10 gelangen kann respektive ein schaltbarer Fluidstrom entweder über die Drosseleinrichtung 27 respektive die Strömungsbohrung 29 oder unter Umgehung derselben möglich ist, ist ein Schaltventil 31 vorgesehen, umfassend das Standrohr 10 bzw. eine im Standrohr 10 ausgebildete Bohrung 32, die bei angehobenem Kolben 8 freiliegt, das heißt, dass sich dann der Kolben 8 respektive die Kolbenstange 9 oberhalb der Bohrung 32 befinden. Ab Erreichen einer bestimmten Kolben- oder Kolbenstangenposition verschließt in diesem Fall die Kolbenstange 9 die Bohrung 32. So lange sie noch geöffnet ist, kann Hydraulikfluid aus dem Zylinderraum 3 in das Standrohr 10 eindringen, ein Fluidstrom über die Längsbohrungen 30 unter Umgehung der eigentlichen Drossel 27 in den Aufnahmeraum 17 ist möglich. Mit Verschluss der Bohrung 32 ist das Schaltventil 31 geschlossen. Das Hydraulikfluid kann bei fortgesetzter Absenkbewegung nur noch über die Drosseleinrichtung 27 respektive die Strömungsbohrung 29 mit entsprechendem Strömungswiderstand in den Aufnahmeraum 17 strömen.

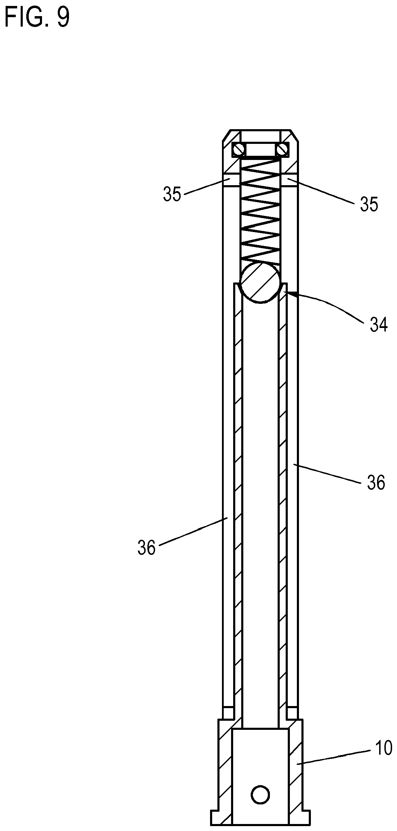

Des Weiteren vorgesehen ist ein zusätzlicher Aufnahmeraum 33 für Hydraulikfluid. Dieser Aufnahmeraum 33 ist im Inneren der Kolbenstange 9 realisiert. Hydraulikfluid kann in diesen Aufnahmeraum 33 über ein im Standrohr 10 integriertes Rückschlagventil 34 gelangen. Dieses Rückschlagventil 34 wird geöffnet, wenn die Kolbenstange 9 aus dem Zylinderraum 3 herausgezogen wird. In diesem Fall strömt, worauf nachfolgend eingegangen wird, aus dem Aufnahmeraum 17 Hydraulikfluid in das Standrohr 10 und öffnet das Rückschlagventil 34. Bei einer entgegengesetzten Bewegung kann das Hydraulikfluid aus dem Aufnahmeraum 33 in den unteren Zylinderraumteil 3a strömen, nachdem am Standrohr 10 - siehe Figur 9 - zwei aneinander gegenüberliegende Durchbrechungen 35 vorgesehen sind, die im Bereich des Rückschlagventils 34 angeordnet sind. An der Außenseite des Standrohres 10 einander gegenüberliegend sind zwei längslaufende Nuten 36 vorgesehen, die an den Durchbrechungen 35 münden. Wird folglich die Kolbenstange 9 nach unten gedrückt, so verkleinert sich der Aufnahmeraum 33. Das darin befindliche Hydraulikfluid strömt sodann über die die Durchbrechungen 35 und die Nuten 36 in den unteren Zylinderraumteil 3a.

Schließlich sind zwei Verstelldrosselventile 37 vorgesehen, die am Zylinderdeckelelement 7 mittels entsprechender Gewindeschrauben 38 realisiert sind. Über sie wird der Strömungsquerschnitt eines zugeordneten Fluidkanals 39, der den oberen Zylinderraumteil 3b mit dem kleineren Aufnahmeraum 21 verbindet, eingestellt. Hierüber wird eine Endlagendämpfung erreicht, worauf nachfolgend noch eingegangen wird. Eines der beiden Verstelldrosselventile 37 ist vom Techniker verstellbar. Hierzu ist es lediglich erforderlich, die Gewindeschraube entsprechend ein- oder auszuschrauben. Das andere Verstelldrosselventil 37 ist werkseitig eingestellt und positionsgesichert, so dass stets ein Mindestdurchfluss gegeben ist, um zu verhindern, dass das Gelenk nicht in vollständige Streckung kommen kann.

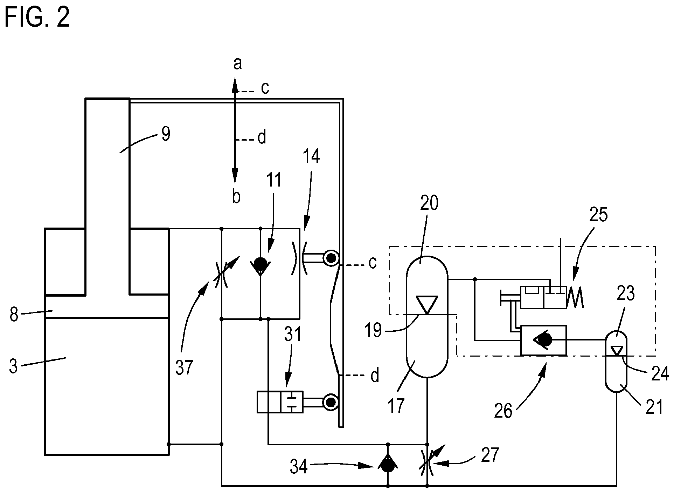

Figur 2 zeigt ein entsprechendes Blockschaltventil des Dämpfungszylinders 1 aus Figur 1. Gezeigt sind die wesentlichen hydraulisch und pneumatisch wirksamen Elemente. Zylinderseitig dargestellt ist der Zylinderraum 3, der Kolben 8 und die Kolbenstange 9. Gezeigt sind ferner die beiden Aufnahmeräume 17 und 21 mit ihren zugeordneten, zur Pneumatik gehörenden Kompressionsräumen 20 und 23, getrennt über die jeweiligen Membranen 19 und 24. Dargestellt ist des Weiteren die Drosseleinrichtung 27, die im Zustrom zum größeren Aufnahmeraum 17 angeordnet ist, während eine solche Drossel oder dergleichen im Zustrom zum kleineren Aufnahmeraum 21 nicht vorgesehen ist.

Ferner ist exemplarisch ein Verstelldrosselventil 37 gezeigt, weiterhin das kolbenseitige Rückschlagventil 11 sowie das Drosselventil 14, über welche Ventile die oberen und unteren Zylinderraumteile 3a, 3b je nach Bewegungsrichtung des Kolbens 8 respektive der Kolbenstange 9 miteinander kommunizieren können.

Weiterhin dargestellt ist das wichtige Schaltventil 31, über das je nach Kolben- / Kolbenstangenposition eine Umgehung der Drosseleinrichtung 27 möglich ist.

Des Weiteren ist, wie durch die strichpunktierte Linie dargestellt ist, der Pneumatikteil gezeigt, umfassend die bereits beschriebenen Kompressionsräume 20 und 23 sowie die Fluidzuführöffnung 25, also den bereits beschriebenen Füllstutzen mit dem 2/2-Wege Ventil. Gezeigt ist schließlich auch das Rückschlagventil 26, gebildet aus dem in Figur 1 gezeigten Stempel 40, der im gezeigten Beispiel gegen zwei elastische Ringe 41 angefedert ist, die den Öffnungswiderstand bilden.

Unterschieden wird zwischen einer hydraulischen Seite und einer pneumatischen Seite. Die hydraulische Seite umfasst sämtliche Volumina, die mit Hydrauliköl gefüllt sind. Dies sind der Zylinderraum 3, sämtliche Hydraulikfluidkanäle, die Aufnahmeräume 17 und 21, das Innere des Standrohres 10 sowie das Innere der Kolbenstange 9, also der zusätzliche Aufnahmeraum 33. Als Hydraulikfluid wird üblicherweise ein Öl verwendet.

Die pneumatische Seite umfasst die beiden Kompressionsräume 20 und 23 sowie den Bereich der Fluidzuführöffnung, soweit dieser nicht dem Kompressionsraum 20 bereits zuzurechnen ist. Diese pneumatischen Bereiche sind mit einem kompressiblen Fluid, wie bereits beschrieben, üblicherweise Luft oder ein anderes Gas, gefüllt.

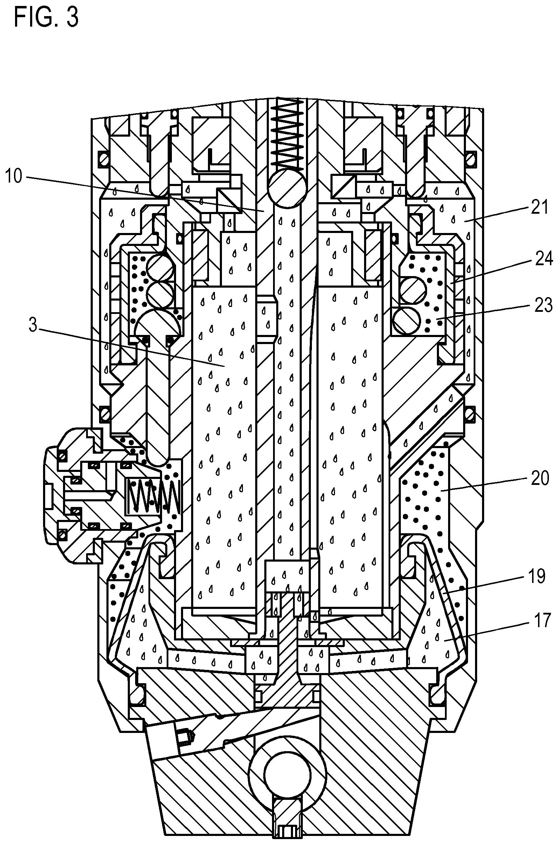

Exemplarisch sind in Figur 3 in einer Teilansicht des Dämpfungszylinders 1 aus Figur 1 die mit dem Hydraulikfluid gefüllten Bereiche entsprechend mit einem Tröpfchensymbol dargestellt, während die mit dem Kompressionsfluid gefüllten Bereiche entsprechend gepunktet dargestellt sind. Wenngleich hier nicht näher gezeigt, ist Hydraulikfluid selbstverständlich auch im Inneren der nicht ganz gezeigten Kolbenstange 9 vorhanden.

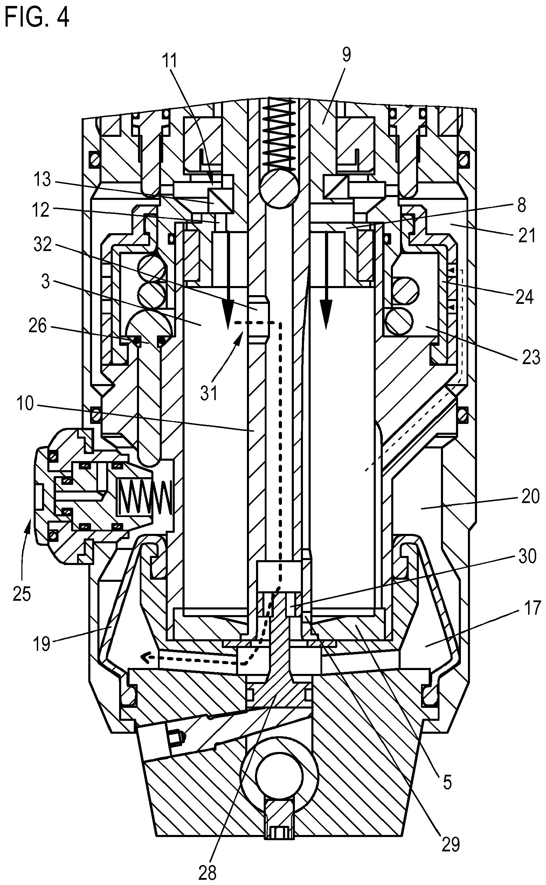

Anhand der Figuren 4 - 8 wird nun das Funktionsprinzip des Dämpfungszylinders 1 beschrieben. Die Kompressionsräume 20 und 23 sind über die Fluidzuführeinrichtung 25 mit dem Kompressionsfluid befüllt. In beiden ist, nachdem ein Ausgleich über das Rückschlagventil 26 möglich ist, der gleiche Druck vorhanden. Die Bewegungsrichtung des Kolbens ist über jeweilige Pfeile, die nach unten oder oben zeigen, angegeben. Der Hydraulikfluidfluss ist durch gestrichelte Pfeillinien dargestellt, wobei eine dickere Pfeillinie einen größeren Fluidfluss als eine dünnere Pfeillinie angibt.

Figur 4 zeigt eine Teilansicht des Dämpfungszylinders, bei der der Kolben 8 und die Kolbenstange 9 in einer herausgezogenen, im Endanschlag befindlichen Position sind. Das Prothesenkniegelenk, in das der Dämpfungszylinder 1 eingebaut ist, ist folglich gestreckt. Der Prothesenträger beginnt nun damit, das Prothesenkniegelenk zu beugen, also die Flexion einzuleiten. Dies führt dazu, dass die Kolbenstange 9 in den Zylinderraum 3 gedrückt wird. Gleichzeitig wird natürlich auch der Kolben 8 nach unten in Richtung des Zylinderbodens 5 bewegt. Diese Bewegung ist durch die beiden dicken Pfeile in Figur 4 gezeigt. Bei dieser Bewegung öffnet sich das Rückschlagventil 11, das heißt, dass der Dichtring 13 aus seinem Dichtsitz am Kolben 8 gehoben wird und die Bohrung 12 offen ist. Der Kolben 8 ist folglich von der Kolbenstange 9 hydraulisch entkoppelt. Dies führt dazu, dass das einzige verdrängende und in den Zylinderraum 3 eintauchende Element die Kolbenstange 9 ist. Durch das Eintauchen der Kolbenstange 9 wird das Hydraulikfluid verdrängt. Das verdrängte Hydraulikfluid strömt in die entsprechenden Aufnahmeräume 17 und 21, wie nachfolgend noch beschrieben wird.

Zu Beginn der Flexionsbewegung ist das Schaltventil 31 geöffnet, das heißt, dass die Bohrung 32 durch die Kolbenstange 9 nicht verschlossen ist. Das ermöglicht es, wie durch die dickere gestrichelte Pfeillinie dargestellt ist, dass Hydraulikfluid in das Innere des Standrohres 10 strömen kann. Es strömt durch die Längsbohrungen 30 im Drosselelement 28 und gelangt somit unter Umgehung der eigentlichen Drossel 27, also der im Strömungsquerschnitt eingestellten Strömungsbohrung 29 in den Aufnahmeraum 17. Es ist also eine Umströmung der Drossel 27 gegeben, dem Hydraulikfluid wird kein nennenswerter Strömungswiderstand entgegengesetzt.

Der überwiegende Hydraulikfluidanteil strömt in den größeren Aufnahmeraum 17. Ein geringer Teil strömt, wie durch die gestrichelten dünneren Strömungspfeile dargestellt ist, in den kleineren Aufnahmeraum 21. Dies deshalb, da der Membranwiderstand, den die flexible Membran 24 dem Hydraulikfluid entgegensetzt, deutlich größer ist als der Membranwiderstand, den die Membran 19 entgegensetzt. Ein Strömungswiderstand über die Drosseleinrichtung 27 ist in diesem Fall, da sie letztlich umgangen wird, nicht gegeben. Aus diesem Grund, da der Hauptfluidstrom in den größeren Aufnahmeraum 17 erfolgt, sind die entsprechenden Strömungspfeile dorthin auch deutlich dicker als die in den kleineren Aufnahmeraum 21 führenden Strömungspfeile.

Die Umströmung der Drosseleinrichtung 27 ist nur bis zu einem gewissen Flexionsgrad möglich, beispielsweise bis zu 30°. Bis zu einer 30°-Beugung ist folglich dem Hydraulikfluid kein nennenswerter Strömungswiderstand beim Einströmen in den großen Aufnahmeraum 17 entgegengesetzt, damit ist der Hydraulikfluidstrom unabhängig von der Geschwindigkeit, mit der die Flexion erfolgt.

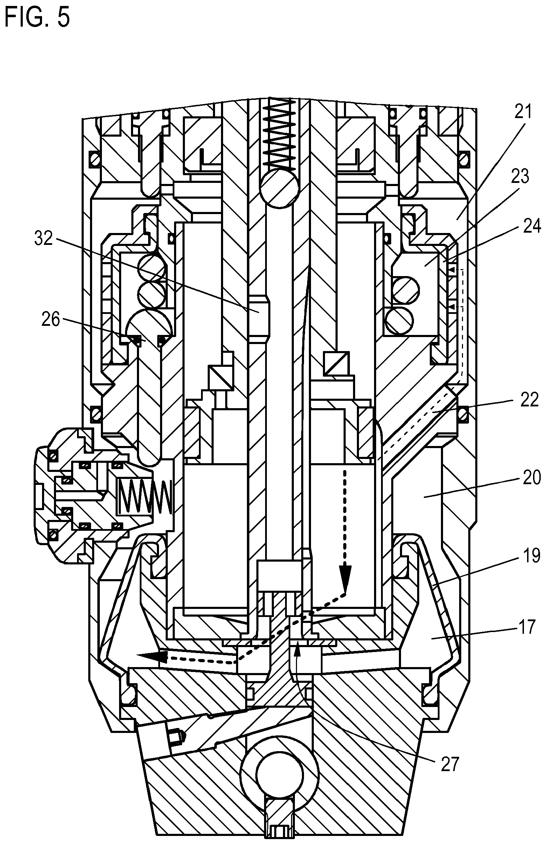

Ab einer Flexion von den exemplarisch beschriebenen 30° jedoch ist, da die Kolbenstange 9 noch tiefer in den Zylinderraum 3 bewegt wird, das Schaltventil 31 geschlossen, das heißt, dass die Bohrung 32 geschlossen ist, siehe hierzu Figur 5. Ein weiterer Hydraulikfluidfluss in das Standrohrinnere ist nicht möglich, das Hydraulikfluid muss zwangsläufig über die Drosseleinrichtung 27, also über die Strömungsbohrung 29 strömen, um in den größeren Aufnahmeraum 17 zu gelangen. Ab diesem Zeitpunkt, also unmittelbar mit dem Verschließen der Bohrung 32 setzt die geschwindigkeitsabhängige Adaption ein. Denn von nun an ist die Verteilung des aus dem Zylinderraum 3 herausgedrückten Hydraulikfluids davon abhängig, wie schnell die Flexionsbewegung ist.

Figur 5 zeigt den Fall, dass die Flexionsbewegung langsam von statten geht. Ab der beschriebenen 30° Flexion kann das Hydraulikfluid nur noch gegen den relativ großen Membranwiderstand der Membran 24, die dem kleineren Aufnahmeraum 21 zugeordnet ist, oder gegen den Strömungswiderstand der Drosseleinrichtung 27 strömen. Bei einer niedrigeren Flexionsgeschwindigkeit ist der Strömungswiderstand der Drosseleinrichtung 27 geringer als der Membranwiderstand der Membran 24 des kleineren Aufnahmeraums. Daher strömt das Hydraulikfluid bevorzugt in den größeren Aufnahmeraum 17. Hierdurch steigt der dortige Druck, es kommt zu einer Deformation der Membran 19 und damit zu einer leichten Druckerhöhung im unteren Kompressionsraum 20. Nur ein geringer Anteil strömt in den kleineren Aufnahmeraum 21, wie durch die wiederum unterschiedlich dicken, gestrichelten Strömungspfeillinien dargestellt ist. Für den Fall, dass der Druck im Kompressionsraum 20 größer wird als der Druck im Kompressionsraum 23, kann ein Druckausgleich über das Rückschlagventil 26 erfolgen.

Je weiter die Drosseleinrichtung 27 geschlossen wird, mithin also der Strömungswiderstand der Drosseleinrichtung 27 vergrößert wird, umso mehr Hydraulikfluid strömt in den kleineren Aufnahmeraum 21, da sich das Verhältnis aus Drossel- respektive Strömungswiderstand und Membranwiderstand ändert. Je mehr Hydraulikfluid jedoch in den kleineren Aufnahmeraum 21 strömt, umso mehr Energie, die nachfolgend für die Extensionsbewegung zurückgewonnen werden kann, wird im Kompressionsfluid im Kompressionsraum 23 gespeichert. Bei sehr langsamer Flexionsbewegung jedoch nimmt der Druck in beiden Aufnahmeräumen 17 und 21 quasi synchron zu, so dass - auch verbunden aufgrund der Druckausgleichsmöglichkeit über das Rückschlagventil 26 - in den Kompressionsräumen 20 und 23 ein vergleichbarer Druck gegeben ist.

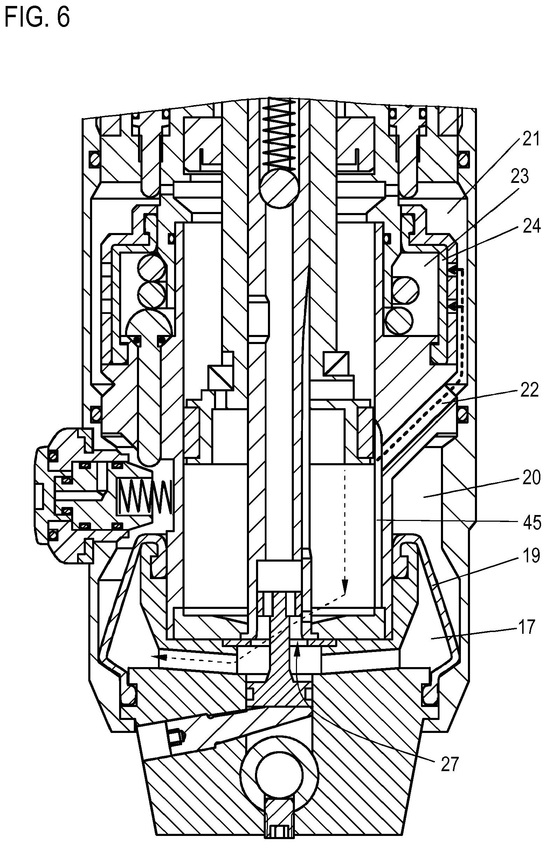

Figur 6 zeigt hingegen die Situation, wenn die Flexion bei geschlossenem Schaltventil 31 schnell respektive sehr schnell erfolgt. Bei hoher Flexionsgeschwindigkeit und damit hoher Eintauchgeschwindigkeit der Kolbenstange 9 - die nach wie vor aufgrund der hydraulischen Entkopplung des Kolbens 8 von der Kolbenstange 9 das einzige Verdrängerelement ist - wird der Strömungswiderstand der Drosseleinrichtung 27 deutlich höher als der Membranwiderstand der Membran 24 des kleineren Aufnahmeraums 21. Damit nimmt geschwindigkeitsabhängig und mit steigender Geschwindigkeit immer stärker der Hydraulikfluidanteil, der in den kleineren Aufnahmeraum 21 strömt, zu, verglichen mit dem geringen Anteil, der noch über die Drosseleinrichtung 27 in den größeren Aufnahmeraum 17 strömt. Es kommt zu einer starken Deformation der Membran 24 und damit zu einer starken Kompression des Kompressionsfluids im Kompressionsraum 23. Hieraus resultiert ein hoher Energieeintrag, das heißt, dass viel Energie im Kompressionsfluid gespeichert wird. Ein Druckausgleich zwischen den Kompressionsräumen 20 und 23 ist in diesem Fall nicht möglich, da das Rückschlagventil in dieser Richtung geschlossen ist. Bei sehr schneller Flexionsbewegung ist der Strömungswiderstand der Drosseleinrichtung 27 so groß, dass das Hydraulikfluid nahezu ausschließlich in den kleineren Aufnahmeraum 21 strömt. Ein Zustrom in den kleineren Aufnahmeraum 21 ist auch beim weiter abgesenktem Kolben 8 über den Fluidkanal 22 jederzeit möglich, da das Hydraulikfluid über eine an der Innenseite des Einbauteils 4 vorgesehene Nut 45, die am Fluidkanal 22 mündet, stets zum Fluidkanal 22 gelangt.

Somit bestimmt ersichtlich die Flexionsgeschwindigkeit die Verteilung des Hydraulikfluids auf die beiden Aufnahmeräume 17 und 21 und damit die Energiemenge, die letztendlich für die nachfolgende Extensionsbewegung gespeichert werden kann. Je höher die Flexionsgeschwindigkeit, umso höher die im Kompressionsfluid, insbesondere im Kompressionsraum 23 gespeicherte Energie und umgekehrt.

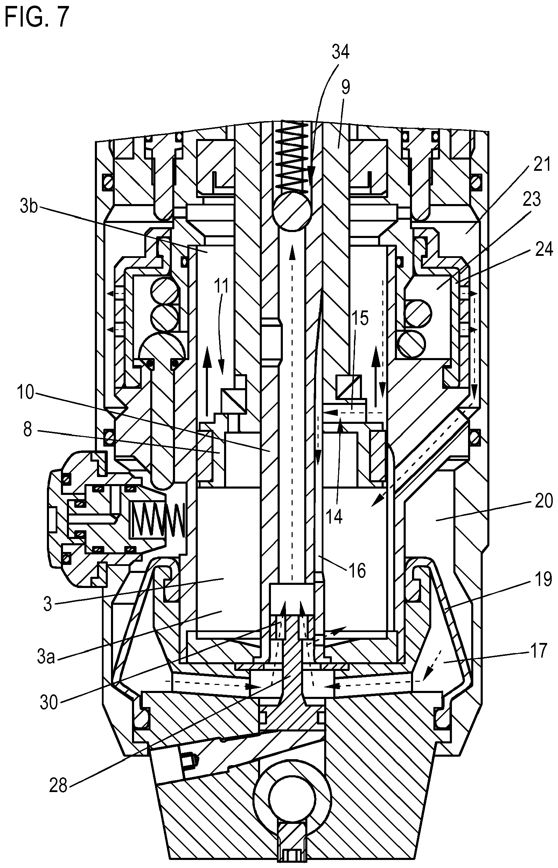

Die Verdrängung des Hydraulikfluids aus dem Zylinderraum 3 erfolgt so lange, bis die Flexion, also die Gelenkbeugung beendet ist. Es schließt sich nun die Extension, also die Gelenkstreckung an. Diese Situation ist zu Beginn der Extensionsbewegung in Figur 7 gezeigt.

Bei der Extensionsbewegung wird die Kolbenstange 9 nebst Kolben 8 wieder aus dem Zylinderraum 3 herausgezogen, sie bewegen sich in Figur 7 gesehen nach oben, wie durch die beiden Bewegungspfeile dargestellt ist.

Die in Fig. 7 gezeigte Situation ergibt sich zu Beginn einer Extensionsbewegung, die einer vorherigen langsamen Flexion folgt, im Rahmen welcher sich der Druck in den beiden Kompressionsräumen 20 und 23 gleichmäßig erhöht hat und folglich gleich ist. Das vorher verdrängte Hydraulikfluid strömt in diesem Fall aus den beiden Aufnahmeräumen 17 und 21 zurück in den Zylinderraum 3 bzw. hier den unteren Zylinderraumteil 3a. Dies ist durch die entsprechend gestrichelten Strömungspfeillinien dargestellt. Getrieben wird dieser Rückstrom jeweils durch die komprimierten Kompressionsfluide in den Kompressionsräumen 20 und insbesondere 23. Sind bei langsamer Flexion die Drücke in beiden Kompressionsräumen 20 und 23 näherungsweise gleich groß, so ist nur eine geringe Extensionsunterstützung gegeben.

Erfolgte zuvor eine schnelle Flexion, so ist ein Druckunterschied zwischen den beiden Kompressionsräumen gegeben, der je nach Flexionsbewegung mehrere bar bis mehrere 10 bar betragen kann. Je mehr Hydraulikfluid in den kleineren Aufnahmeraum 21 geströmt ist, umso größer ist die im Kompressionsraum 23 gespeicherte Energiemenge, die nun zurückgewonnen werden kann. Die flexible Membran 24 drückt mit hohem Druck auf das Hydraulikfluid und drückt dieses entsprechend kräftig aus dem kleineren Aufnahmeraum 21 in den Zylinderraum 3 zurück. Umso größer respektive druckvoller dieser Rückstrom ist, umso stärker ist die Unterstützungskraft im Rahmen der Extensionsbewegung, das heißt, umso stärker ist die Winkelbeschleunigung zu Beginn der Extensionsbewegung. Das aus dem kleineren Aufnahmeraum mit hohem Druck zurückströmende Fluid in den Zylinderraumteil 3a strömt aufgrund des gegebenen Druckunterschieds jedoch auch noch über die Drosseleinrichtung 27 in den großen Aufnahmeraum, dieser füllt sich also noch weiter, verbunden mit einer leichten Druckerhöhung im zugeordneten Kompressionsraum 20. D.h., dass in diesem Fall - anders als in Fig. 7 gezeigt - die unteren Strömungspfeile zum größeren Aufnahmeraum 17 zeigen. Dies geschieht solange, bis ein Druckausgleich gegeben ist. Danach strömt bei fortgesetzter Extensionsbewegung das Hydraulikfluid, wie in Fig. 7 gezeigt, aus beiden Aufnahmeräumen in den Zylinderraumteil 3a. Die dann noch gegebene Extensionsunterstützung hängt von der verbleibenden, noch gespeicherten Restenergie in den beiden Kompressionsräumen 20 und 23 ab.

Das Drosselventil 14 ist, siehe Figur 7, nach wie vor offen, da sich die Bohrung 15 noch benachbart zur Nut 16 befindet. Das heißt, dass Hydraulikfluid aus dem aufgrund der Herausziehbewegung des Kolbens und der Kolbenstange verkleinernden oberen Zylinderraum 3b über das Drosselventil 14 in den unteren Zylinderraum 3a strömen kann. Nach wie vor sind der Kolben 8 und die Kolbenstange 9 miteinander hydraulisch entkoppelt, weshalb wiederum nur die Kolbenstangenfläche das Verdrängerelement ist. Gleichzeitig erhöht sich, nachdem aus dem größeren Aufnahmeraum 17 das Hydraulikfluid, wie Figur 7 zeigt, durch das Drosselelement 28 respektive die dortigen Längsbohrungen 30 in das Innere des Standrohes 10 einströmt, und nachdem das Schaltventil 31 nach wie vor geschlossen ist, der Druck im Standrohrinneren. Das Rückschlagventil 34 öffnet nun druckbedingt, so dass das Hydraulikfluid auch in den zusätzlichen Aufnahmeraum 33 in der Kolbenstange 9 strömen kann. Das Rückschlagventil 11 ist während der gesamten Extensionsbewegung geschlossen.

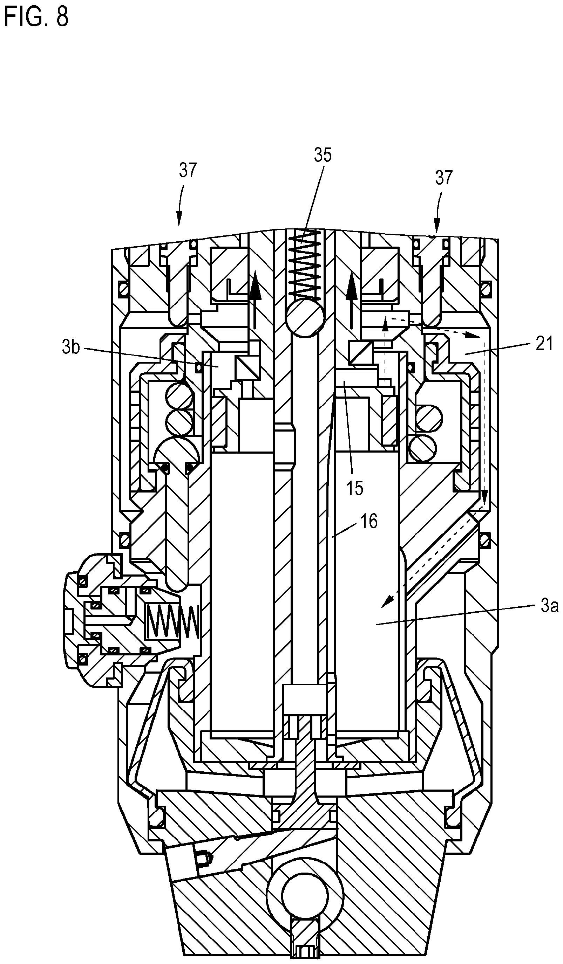

Je nach gespeichertem Energieeintrag ist folglich eine schnelle Extensionsbewegung respektive eine starke Bewegungsunterstützung durch die rückgewonnene Energie möglich. Dies so lange, so lange das Drosselventil 14 geöffnet ist. Wie beschrieben läuft die Nut 16 im Bereich ihres obigen Endes langsam aber stetig aus, geht also auf null. Ist nun die Kolbenstange 9 hinreichend weit herausgezogen, so schließt sich langsam auch der Strömungsquerschnitt der Bohrung 15, so dass mit Erreichen einer bestimmten Position, beispielsweise einem noch gegebenen Beugungsgrad von 15°, das Drosselventil 14 geschlossen ist. Bei einer weiteren Bewegung, wie in Figur 8 durch die beiden Bewegungspfeile dargestellt ist, muss nun das Hydraulikfluid aus dem sich weiterhin verkleinernden Zylinderraum 3b respektive dem noch verbliebenen Restvolumen oberhalb des Kolbens 8 über die beiden Verstelldrosselventile 37 in den kleineren Aufnahmeraum 21 und über diesen in den unteren Zylinderraumteil 3a strömen. Da das Drosselventil 14 und das Rückschlagventil 11 nun geschlossen ist, sind Kolben 8 und Kolbenstange 9 nunmehr hydraulisch gekoppelt, das heißt, dass die hydraulische Wirkfläche jetzt auch die Kolbenfläche einschließt.

Wurde über die auslaufende Nut 16 die Extensionsbewegung zu ihrem Ende hin bereits gedämpft, da sich der freie Strömungsquerschnitt der Bohrung 15 langsam verschließt, so ist über die Verstelldrosselventile 37 eine Endlagendämpfung realisiert. Denn die Verstelldrosselventile 37 lassen nur einen äußerst geringen Durchfluss zu, so dass nur noch eine geringe, stark gedämpfte Bewegung des Kolbens 8 in seine Endlage möglich ist. Wie beschrieben ist eines der Verstelldrosselventile 37 positionsfest und stellt einen Mindestdurchfluss sicher, während das Andere zur Einstellung der Endlagendämpfung verstellbar ist.

Mit Erreichen der Endlage ist die Extensionsbewegung abgeschlossen. Es setzt nun eine erneute Flexionsbewegung ein beginnend mit der Situation gemäß Figur 4 und der Bewegung des Kolbens 8 nebst Kolbenstange 9 nach unten.

Mit Einsetzen dieser Bewegung wird das Hydraulikfluid wieder aus dem Zylinderraum 3 in der beschriebenen Weise verdrängt. Gleichzeitig wird aber auch aus dem zusätzlichen Aufnahmeraum 33 das Hydraulikfluid in den unteren Zylinderrumteil 3a gefördert. Das Hydraulikfluid strömt über die insbesondere in Figur 9 deutlich gezeigte Durchbrechung 35, von denen zwei einander gegenüberliegend vorgesehen sind und die hier nicht gezeigte, jedoch auch Figur 9 entnehmbare Längsnut 36 nach unten in den unteren Zylinderraumteil 3a.

## Patentansprüche

1. Hydraulischer Dämpfungszylinder, insbesondere für ein Prothesenkniegelenk, umfassend ein Gehäuse, einen im Gehäuse vorgesehenen und mit einem Hydraulikfluid gefüllten Zylinderraum, und einen in diesem angeordneten Kolben, der über eine in den Zylinderraum eingeführte Kolbenstange im Zylinderraum beweglich ist, wobei im Gehäuse (2) wenigstens zwei separate, unterschiedlich große, mit dem Zylinderraum (3) über Fluidkanäle verbundene Aufnahmeräume (17, 21) für bei einer Kolbenbewegung aus dem Zylinderraum (3) verdrängtes Hydraulikfluid vorgesehen sind, die jeweils über eine Membran (19, 24) von einem mit einem kompressiblen, einen Energiespeicher bildenden Fluid gefüllten Kompressionsraum (20, 23) getrennt sind, wobei dem größeren Aufnahmeraum (17) eine einen Strömungswiderstand für das in den Aufnahmeraum (17) strömende Hydraulikfluid bildende Drosseleinrichtung (27) vorgeschaltet ist, derart, dass das Hydraulikfluid auf die beiden Aufnahmeräume (17, 21) in Abhängigkeit der Geschwindigkeit der Kolbenbewegung verteilbar ist.

2. Dämpfungszylinder nach Anspruch 1, **dadurch gekennzeichnet, dass** der Zylinderraum (3) durch ein im Gehäuse angeordnetes Einsatzbauteil (4) ausgebildet ist.

3. Dämpfungszylinder nach Anspruch 1 oder 2, **dadurch gekennzeichnet, dass** der Strömungsquerschnitt der Drosseleinrichtung (27) einstellbar ist.

4. Dämpfungszylinder nach Anspruch 3, **dadurch gekennzeichnet, dass** die Drosseleinrichtung (27) ein verstellbares Drosselelement (28) umfasst, über das der Querschnitt einer zum Aufnahmeraum (17) führenden Strömungsbohrung (29) variierbar ist.

5. Dämpfungszylinder nach einem der vorangehenden Ansprüche, **dadurch gekennzeichnet, dass** die Drosseleinrichtung (27) im Bereich des Bodens (5) des Zylinderraums (3) vorgesehen ist, wobei auch der größere Aufnahmeraum (17) im Bereich des Zylinderbodens (5), diesen zumindest abschnittsweise umgebend, vorgesehen ist, während der kleinere Aufnahmeraum (21) oberhalb davon, insbesondere im Bereich des Zylinderdeckelelements (7) vorgesehen ist.

6. Dämpfungszylinder nach einem der vorangehenden Ansprüche, **dadurch gekennzeichnet, dass** eine reversibel verschließbare Fluidzuführöffnung (25) vorgesehen ist, über die kompressibles Fluid in Bezug auf die Kompressionsräume (20, 23) einfüll- oder ablassbar ist.

7. Dämpfungszylinder nach Anspruch 6, **dadurch gekennzeichnet, dass** die beiden Kompressionsräume (20, 23) über ein Rückschlagventil (26) miteinander verbunden sind, das bei einem Überdruck im dem größeren Aufnahmeraum (17) zugeordneten Kompressionsraum (20) öffnet.

8. Dämpfungszylinder nach einem der vorangehenden Ansprüche, **dadurch gekennzeichnet, dass** die jeweilige Membran (19, 24) eine Gummi- oder Kunststoffmembran ist.

9. Dämpfungszylinder nach einem der vorangehenden Ansprüche, **dadurch gekennzeichnet, dass** den Aufnahmeräumen (17, 21) ein Schaltventil (31) vorgeschaltet ist, über das der Hydraulikfluidstrom in Abhängigkeit der Position des Kolbens (8) oder der Kolbenstange (9) dem größeren Aufnahmeraum (17) an der Drosseleinrichtung (27) vorbei oder über die Drosseleinrichtung (27) zuführbar ist.

10. Dämpfungszylinder nach Anspruch 9 **dadurch gekennzeichnet, dass** das Schaltventil (31) ein am Zylinderboden (5) aufstehendes Standrohr (10) umfasst, das vom Kolben (8) und von der Kolbenstange (9) übergriffen ist, und das über eine Bohrung (32) mit dem Zylinderraum (3) kommuniziert, so dass Hydraulikfluid durch das Standrohr (10) solange in den größeren Aufnahmeraum (17) zuführbar ist, bis die Bohrung (32) durch den in Richtung des Zylinderbodens (5) bewegten Kolben (8) oder die Kolbenstange (9) geschlossen ist, wonach das Hydraulikfluid über die Drosseleinrichtung (27) strömt.

11. Dämpfungszylinder nach Anspruch 10, **dadurch gekennzeichnet, dass** das Drosselelement (28) in das Standrohr (10) eingreift und die Strömungsbohrung (29) im Standrohr (10) ausgebildet ist.

12. Dämpfungszylinder nach einem der vorangehenden Ansprüche, **dadurch gekennzeichnet, dass** am Kolben (8) ein bei einer Bewegung in Richtung des Zylinderbodens (5) öffnendes Rückschlagventil (11) vorgesehen ist, über das der unterhalb des Kolbens (8) befindliche Teil (3a) des Zylinderraums (3) mit dem oberhalb des Kolbens (8) befindlichen Teil (3b) des Zylinderraums (3) verbindbar ist.

13. Dämpfungszylinder nach Anspruch 12, **dadurch gekennzeichnet, dass** das Rückschlagventil (11) einen die Kolbenstange (9) umgreifenden Dichtring (13) und eine am Kolben (8) vorgesehene Bohrung (12), die vom Dichtring (13) übergriffen ist, umfasst.

14. Dämpfungszylinder nach einem der vorangehenden Ansprüche, **dadurch gekennzeichnet, dass** ein Drosselventil (14) vorgesehen ist, über das der unterhalb des Kolbens (8) befindliche Teil (3a) des Zylinderraums (3) mit dem oberhalb des Kolbens (8) befindlichen Teil (3b) des Zylinderraums (3) kommuniziert, über welches Drosselventil (14) bei einer Bewegung des Kolbens (8) in Richtung des Zylinderdeckelelements (7) Hydraulikfluid aus dem oberhalb des Kolbens (8) befindlichen Teil (3b) in den unterhalb befindlichen Teil (3a) strömt, bis es mit Erreichen einer definierten Kolbenposition geschlossen ist.

15. Dämpfungszylinder nach Anspruch 14, **dadurch gekennzeichnet, dass** das Drosselventil (14) eine am Standrohr (10) längslaufende Nut (16) und eine kolben- oder kolbenstangenseitig ausgebildete Bohrung (15) umfasst, die bei geöffnetem Drosselventil (14) mit der Nut (16) und dem oberen Teil (3b) des Zylinderraums (3) kommuniziert.

16. Dämpfungszylinder nach einem der vorangehenden Ansprüche, **dadurch gekennzeichnet, dass** wenigstens ein Verstelldrosselventil (37) vorgesehen ist, über das der Strömungsquerschnitt eines Hydraulikfluidkanals (39), der den oberhalb des Kolbens (8) befindlichen Teil (3b) des Zylinderraums (3) mit dem kleineren Ausnahmeraum (21) verbindet, variierbar ist.

17. Dämpfungszylinder nach Anspruch 16, **dadurch gekennzeichnet, dass** das Verstelldrosselventil (37) eine über ein Gewinde im Gehäuse (2) beweglich geführte Drosselschraube (38) umfasst, über die der Querschnitt des Hydraulikfluidkanals (39) variierbar ist.

18. Dämpfungszylinder nach Anspruch 16 oder 17, **dadurch gekennzeichnet, dass** ein zweites Verstelldrosselventil (37) vorgesehen ist, das in einer festen Stellung, einen Mindestdurchfluss definierend, gesichert, insbesondere verplombt ist.

19. Dämpfungszylinder nach Anspruch 10 **dadurch gekennzeichnet, dass** ein zusätzlicher Aufnahmeraum (33) für bei einer Bewegung des Kolbens (8) in Richtung des Zylinderdeckelelements (7) aus dem größeren Aufnahmeraum (17) zurückströmendes Hydraulikfluid vorgesehen ist.

20. Dämpfungszylinder nach Anspruch 19, **dadurch gekennzeichnet, dass** der zusätzliche Aufnahmeraum (33) im Inneren der Kolbenstange (9) vorgesehen ist, die über ein Rückschlagventil (34) mit dem Inneren des Standrohres (10) verbunden ist, welches Rückschlagventil (34) bei dieser Bewegung durch den anliegenden Hydraulikfluiddruck des vom größeren Aufnahmeraum (17) her in das Standrohr (10) einströmenden Hydraulikfluids öffnet.

21. Dämpfungszylinder nach Anspruch 20, **dadurch gekennzeichnet, dass** am Standrohr (10) wenigstens eine im Bereich des oder oberhalb des Rückschlagventils (34) angeordnete Durchbrechung (35) vorgesehen ist, an der eine an der Außenseite des Standrohr (10) ausgebildete, sich in Richtung des Zylinderbodens (5) erstreckende Nut (36) mündet.

22. Prothesenkniegelenk, umfassend einen Dämpfungszylinder (1) nach einem der vorangehenden Ansprüche.

## Claims

1. A hydraulic damping cylinder, in particular for a prosthetic knee joint, comprising a housing, a cylinder chamber provided in the housing and filled with a hydraulic fluid, and a piston arranged in said cylinder chamber, the piston being movable in the cylinder chamber via a piston rod inserted in the cylinder chamber, wherein in the housing (2) there are provided at least two separate, differently sized receiving chambers (17, 21) that are connected with the cylinder chamber (3) via fluid channels, said receiving chambers being provided for hydraulic fluid displaced from the cylinder chamber (3) upon a piston movement and each being separated through a membrane (19, 24) from a compression chamber (20, 23) filled with a compressible fluid forming an energy storage, wherein a throttle device (27) forming a flow resistance for the hydraulic fluid flowing into the receiving chamber (17) is disposed upstream of the larger receiving chamber (17), such that the hydraulic fluid can be distributed to the two receiving chambers (17, 21) in dependence on the speed of the piston movement.

2. The damping cylinder according to claim 1, **characterized in that** the cylinder chamber (3) is formed by an insert component (4) arranged in the housing.

3. The damping cylinder according to claim 1 or 2, **characterized in that** the flow cross section of the throttle device (27) is adjustable.

4. The damping cylinder according to claim 3, **characterized in that** the throttle device (27) comprises an adjustable throttle element (28) through which the cross section of a flow bore (29) leading to the receiving chamber (17) is variable.

5. The damping cylinder according to any of the preceding claims, **characterized in that** the throttle device (27) is provided in the region of the bottom (5) of the cylinder chamber (3), wherein the larger receiving chamber (17) is likewise provided in the region of the cylinder bottom (5), surrounding the same at least regionally, while the smaller receiving chamber (21) is provided thereabove, in particular in the region of the cylinder cover element (7).

6. The damping cylinder according to any of the preceding claims, **characterized in that** a reversibly closable fluid supply opening (25) is provided, via which compressible fluid can be supplied or discharged with reference to the compression chambers (20, 23)

7. The damping cylinder according to claim 6, **characterized in that** the two compression chambers (20, 23) are interconnected via a check valve (26) which opens upon overpressure in the compression chamber (20) allocated to the larger receiving chamber (17).

8. The damping cylinder according to any of the preceding claims, **characterized in that** the respective membrane (19, 24) is a rubber or plastic membrane.

9. The damping cylinder according to any of the preceding claims, **characterized in that** upstream of the receiving chambers (17, 21) a switching valve (31) is disposed, via which the flow of hydraulic fluid can be supplied to the larger receiving chamber (17) bypassing the throttle device (27) or via the throttle device (27) in dependence on the position of the piston (8) or the piston rod (9).

10. The damping cylinder according to claim 9, **characterized in that** the switching valve (31) comprises a standpipe (10) standing up on the cylinder bottom (5), said standpipe being straddled by the piston (8) and by the piston rod (9), and communicating with the cylinder chamber (3) via a bore (32), so that hydraulic fluid can be supplied through the standpipe (10) to the larger receiving chamber (17) for such a time until the bore (32) is closed by the piston (8) or the piston rod (9) moved in the direction of the cylinder bottom (5), after which the hydraulic fluid flows via the throttle device (27).

11. The damping cylinder according to claim 10, **characterized in that** the throttle element (28) engages in the standpipe (10) and the flow bore (29) is formed in the standpipe (10).

12. The damping cylinder according to any of the preceding claims, **characterized in that** on the piston (8) a check valve (11) is provided which opens upon a movement in the direction of the cylinder bottom (5), via which check valve the part (3 a) of the cylinder chamber (3) disposed below the piston (8) can be connected with the part (3b) of the cylinder chamber (3) disposed above the piston (8).

13. The damping cylinder according to claim 12, **characterized in that** the check valve (11) comprises a sealing ring (13) gripping around the piston rod (9) and a bore (12) provided on the piston (8) that is straddled by the sealing ring (13).

14. The damping cylinder according to any of the preceding claims, **characterized in that** a throttle valve (14) is provided, via which the part (3a) of the cylinder chamber (3) disposed below the piston (8) communicates with the part (3b) of the cylinder chamber (3) disposed above the piston (8), via which throttle valve (14), upon movement of the piston (8) in the direction of the cylinder cover member (7), hydraulic fluid flows from the part (3b) disposed above the piston (8) into the part (3a) disposed below until it is closed upon reaching a defined piston position.

15. The damping cylinder according to claim 14, **characterized in that** the throttle valve (14) comprises a longitudinal groove (16) extending on the standpipe (10) and a bore (15) formed on the side of the piston or piston rod, said bore communicating with the groove (16) and the upper part (3b) of the cylinder chamber (3) when the throttle valve (14) is open.

16. The damping cylinder according to any of the preceding claims, **characterized in that** at least one adjusting throttle valve (37) is provided, via which the flow cross-section of a hydraulic fluid channel (39) connecting the part (3b) of the cylinder chamber (3) disposed above the piston (8) with the smaller receiving chamber (21) is variable.

17. The damping cylinder according to claim 16, **characterized in that** the adjusting throttle valve (37) comprises throttle screw (38) movably guided via a thread in the housing (2), through which the cross-section of the hydraulic fluid channel (39) is variable.

18. The damping cylinder according to claim 16 or 17, **characterized in that** a second adjusting throttle valve (37) is provided which is secured, in particular sealed, in a fixed position, defining a minimum flow rate.

19. The damping cylinder according to claim 10, **characterized in that** an additional receiving chamber (33) is provided for hydraulic fluid flowing back from the larger receiving chamber (17) upon movement of the piston (8) in the direction of the cylinder cover element (7).

20. The damping cylinder according to claim 19, **characterized in that** the additional receiving chamber (33) is provided in the interior of the piston rod (9), which is connected with the interior of the standpipe (10) via a check valve (34), which check valve (34) opens upon this movement through the present hydraulic fluid pressure of the hydraulic fluid flowing into the standpipe (10) from the larger receiving chamber (17).

21. The damping cylinder according to claim 20, **characterized in that** on the standpipe (10) there is provided at least one aperture (35) arranged in the region of or above the check valve (34), to which aperture there opens a groove (36) that is formed on the outside of the standpipe (10) and extends in the direction of the cylinder bottom (5).

22. A prosthetic knee joint, comprising a damping cylinder (1) according to any one of the preceding claims.

## Revendications

1. Cylindre hydraulique d'amortissement, en particulier pour une articulation de prothèse du genou, comprenant un boîtier, une chambre de cylindre prévue dans le boîtier et remplie d'un fluide hydraulique, et un piston y étant agencé, lequel est déplaçable dans la chambre de cylindre par l'intermédiaire d'une tige de piston introduite dans la chambre de cylindre, cependant que, dans le boîtier (2), au moins deux logements de réception (17, 21) distincts, de taille différente, reliés à la chambre de cylindre (3) par l'intermédiaire de canaux à fluide, sont prévus pour du fluide hydraulique refoulé de la chambre de cylindre (3) lors d'un mouvement du piston, et sont respectivement, par l'intermédiaire d'une membrane (19, 24), séparés d'une chambre de compression (20, 23) remplie d'un fluide compressible constituant un réservoir d'énergie, cependant que, en amont du logement de réception (17) de plus grande taille, un dispositif d'étranglement (27) constituant une résistance à l'écoulement du fluide hydraulique s'écoulant dans le logement de réception (17) est monté, de telle façon que le fluide hydraulique peut être réparti sur les deux logements de réception (17, 21) en fonction de la vitesse du mouvement de piston.

2. Cylindre d'amortissement selon la revendication 1, **caractérisé en ce que** la chambre de cylindre (3) est formée par un composant d'insertion (4) agencé dans le boîtier.

3. Cylindre d'amortissement selon la revendication 1 ou 2, **caractérisé en ce que** la section transversale d'écoulement du dispositif d'étranglement (27) est réglable.

4. Cylindre d'amortissement selon la revendication 3, **caractérisé en ce que** le dispositif d'étranglement (27) comprend un élément d'étranglement (28) ajustable par l'intermédiaire duquel la section transversale d'un alésage d'écoulement (29) conduisant au logement de réception (17) peut être amenée à varier.

5. Cylindre d'amortissement selon une des revendications précédentes, **caractérisé en ce que** le dispositif d'étranglement (27) est prévu dans la zone du fond (5) de la chambre de cylindre (3), cependant qu'aussi le logement de réception (17) de plus grande taille est prévu dans la zone du fond du cylindre (5), entourant ce dernier au moins en certains segments, tandis que le logement de réception (21) de plus faible taille est prévu au-dessus de cela, en particulier dans la zone de l'élément de recouvrement de cylindre (7).

6. Cylindre d'amortissement selon une des revendications précédentes, **caractérisé en ce qu'**un orifice d'amenée de fluide (25) pouvant être réversiblement fermé est prévu, par l'intermédiaire duquel du fluide compressible relativement aux chambres de compression (20, 23) peut être versé ou évacué.

7. Cylindre d'amortissement selon la revendication 6, **caractérisé en ce que** les deux chambres de compression (20, 23) sont reliées l'une à l'autre par l'intermédiaire d'une soupape antiretour (26) qui, en cas de pression excessive dans la chambre de compression (20) affectée au logement de réception (17) de plus grande taille, s'ouvre.

8. Cylindre d'amortissement selon une des revendications précédentes, **caractérisé en ce que** la membrane respective (19, 24) est une membrane en caoutchouc ou en matière plastique.

9. Cylindre d'amortissement selon une des revendications précédentes, **caractérisé en ce que**, en amont du logement de réception (17, 21), une soupape de commutation (31) est montée, par l'intermédiaire de laquelle le flux de fluide hydraulique peut, en fonction de la position du piston (8) ou de la tige de piston (9), être amené au logement de réception (17) de plus grande taille, en passant devant le dispositif d'étranglement (27) ou par l'intermédiaire du dispositif d'étranglement (27).

10. Cylindre d'amortissement selon la revendication 9, **caractérisé en ce que** la soupape de commutation (31) comprend un tube vertical (10) qui est debout au fond du cylindre (5), est enjambé par le piston (8) et par la tige de piston (9), et communique par l'intermédiaire d'un alésage (32) avec la chambre de cylindre (3), de telle sorte que du fluide hydraulique peut être amené à travers le tube vertical (10) dans le logement de réception (17) de plus grande taille jusqu'à ce que l'alésage (32) soit fermé par le piston (8) déplacé en direction du fond du cylindre (5) ou par la tige de piston (9), après quoi le fluide hydraulique s'écoule en passant par le dispositif d'étranglement (27).

11. Cylindre d'amortissement selon la revendication 10, **caractérisé en ce que** l'élément d'étranglement (28) s'engage dans le tube vertical (10) et **en ce que** l'alésage d'écoulement (29) est réalisé dans le tube vertical (10).

12. Cylindre d'amortissement selon une des revendications précédentes, **caractérisé en ce que**, au piston (8), une soupape antiretour (11) s'ouvrant lors d'un mouvement en direction du fond du cylindre (5) est prévue, par l'intermédiaire de laquelle la partie (3a) de la chambre de cylindre (3) qui se trouve au-dessous du piston (8) peut être reliée à la partie (3b) de la chambre de cylindre (3) qui se trouve au-dessus du piston (8).

13. Cylindre d'amortissement selon la revendication 12, **caractérisé en ce que** la soupape antiretour (11) comprend une bague d'étanchéité (13) entourant la tige de piston (9) et un alésage prévu au piston (8) qui est enjambé par la bague d'étanchéité (13).

14. Cylindre d'amortissement selon une des revendications précédentes, **caractérisé en ce qu'**une soupape d'étranglement (14) est prévue, par l'intermédiaire de laquelle la partie (3a) de la chambre de cylindre (3) qui se trouve au-dessous du piston (8) communique avec la partie (3b) de la chambre de cylindre (3) qui se trouve au-dessus du piston (8), soupape d'étranglement (14) par l'intermédiaire de laquelle, lors d'un mouvement du piston (8) en direction de l'élément de recouvrement de cylindre (7), du fluide hydraulique s'écoule depuis la partie (3b) qui se trouve au-dessus du piston (8) vers la partie (3a) qui se trouve au-dessous, jusqu'à ce que, dès qu'une position de piston définie est atteinte, elle soit fermée.

15. Cylindre d'amortissement selon la revendication 14, **caractérisé en ce que** la soupape d'étranglement (14) comprend une rainure (16) longeant le tube vertical (10) et un alésage (15) réalisé du côté du piston (8) ou de la tige de piston (9) qui, quand la soupape d'étranglement (14) est ouverte, communique avec la rainure (16) et avec la partie supérieure (3b) de la chambre de cylindre (3).

16. Cylindre d'amortissement selon une des revendications précédentes, **caractérisé en ce qu'**au moins une soupape d'étranglement réglable (37) est prévue, par l'intermédiaire de laquelle la section transversale d'écoulement d'un canal à fluide hydraulique (39) qui relie la partie (3b) de la chambre de cylindre (3) qui se trouve au-dessus du piston (8) au logement de réception (21) de plus faible taille peut être amenée à varier.

17. Cylindre d'amortissement selon la revendication 16, **caractérisé en ce que** la soupape d'étranglement réglable (37) comprend une vis d'étranglement (38) qui est guidée de manière mobile par l'intermédiaire d'un filetage situé dans le boîtier (2) et par le biais de laquelle la section transversale d'écoulement du canal à fluide hydraulique (39) peut être amenée à varier.

18. Cylindre d'amortissement selon la revendication 16 ou 17, **caractérisé en ce qu'**une deuxième soupape d'étranglement réglable (37) est prévue, laquelle, dans une position fixe définissant un débit minimum, est sécurisée, en particulier plombée.

19. Cylindre d'amortissement selon la revendication 10, **caractérisé en ce qu'**un logement de réception (33) supplémentaire pour du fluide hydraulique refluant du logement de réception (17) de plus grande taille lors d'un mouvement du piston (8) en direction de l'élément de recouvrement de cylindre (7) est prévu.

20. Cylindre d'amortissement selon la revendication 19, **caractérisé en ce que** le logement de réception (33) supplémentaire est prévu à l'intérieur de la tige de piston (9) qui, par l'intermédiaire d'une soupape antiretour (34), est reliée avec l'intérieur du tube vertical (10), ladite soupape antiretour (34) s'ouvrant lors de ce mouvement sous l'effet de la pression de fluide hydraulique exercée par le fluide hydraulique qui s'écoule depuis le logement de réception (17) de plus grande taille jusque dans le tube vertical (10).

21. Cylindre d'amortissement selon la revendication 20, **caractérisé en ce que**, au tube vertical (10), au moins une découpe (35) agencée dans la zone de la soupape antiretour (34) ou au-dessus d'elle est prévue, sur laquelle débouche une rainure (36) qui est réalisée sur la face extérieure du tube vertical (10) et qui s'étend en direction du fond du cylindre (5).

22. Articulation de prothèse du genou, comprenant un cylindre d'amortissement (1) selon une des revendications précédentes.
